(19) Europäisches Patentamt
European Patent Office
Office européen des brevets

(11) EP 1 175 885 A2

(12) # EUROPÄISCHE PATENTANMELDUNG

(43) Veröffentlichungstag:
**30.01.2002 Patentblatt 2002/05**

(51) Int Cl.[7]: **A61K 7/02**, A61K 7/035, A61K 7/48

(21) Anmeldenummer: **01116614.7**

(22) Anmeldetag: **12.07.2001**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **26.07.2000 DE 10036316**

(71) Anmelder: **Beiersdorf Aktiengesellschaft
20245 Hamburg (DE)**

(72) Erfinder:
- **Lanzendörfer, Ghita, Dr.
22087 Hamburg (DE)**
- **Bormann, Angelika
22041 Hamburg (DE)**

(54) **Kosmetische Puderformulierungen**

(57) Kosmetische oder dermatologische Puderzubereitungen, enthaltend

a) Wasser,
b) mindestens ein Hydrokolloid und
c) mindestens ein hydrophobes Pigment.

und Verfahren zu ihrer Herstellung.

EP 1 175 885 A2

**Beschreibung**

Die vorliegende Erfindung betrifft kosmetische Puder, welche Wasser enthalten. Insbesondere betrifft die Erfindung dekorative Puderformulierungen, aber auch desodorierende oder antitranspirierend wirksame Puder sowie Puderformulierungen, welche beispielsweise zur Verwendung gegen Akne geeignet sind und dergleichen mehr.

In der Kosmetik gibt es für Puderformulierungen eine Reihe von Anwendungsgebieten; üblich sind beispielsweise Körper-, Gesichts- und Babypuder sowie Deo- und Fußpuder, aber auch reinigende Pulverformulierungen wie z. B. Puder- bzw. Trockenshampoos.

In klassischen Puderformulierungen liegen ausschließlich Feststoffe nebeneinander vor. Sie werden durch Mischen fester Bestandteile hergestellt und können als lose Puder oder in gepreßter Form als sogenannte Pudersteine vorliegen.

Je nach Anwendungszweck sollen kosmetische Puderformulierungen möglichst die folgenden Eigenschaften besitzen:

- Gesichtspuder:
  Deck- und Haftvermögen bei geringer Öl- und Wasseradsorption
- Körperpuder:
  Glätte und gutes Saugvermögen
- Fußpuder:
  starkes Adsorptionsvermögen, fungizide Wirkung
- Deodorantpuder:
  starkes Adsorptionsvermögen, bakterizide Wirkung

Diese Eigenschaften lassen sich durch Verwendung bestimmter Puderbestandteile erreichen. So werden beispielsweise Siliciumdioxid, Magensiumcarbonat, Kaolin, Kreide oder Stärke eingesetzt, um Puder herstellen, die entweder Wasser und hydrophile Stoffe oder aber Öle, Fette oder lipophile Stoffe aufsaugen können. Titandioxid, Zink-C:\TEMP\Aqua Powder 240700.DOC oxid und Kreide werden verwendet, um die Deckkraft und das Haftvermögen eines Puders zu erhöhen. Das Haftvermögen läßt sich ferner durch Stärke, Aluminiumoxid, Zinkundecanat oder auch Metallseifen verbessern. Desweiteren soll durch den Einsatz von Zink-, Magnesium- und/oder Aluminiumsalzen der Laurin-, Myristin- und/oder Stearinsäure sowie durch Zink- und/oder Magnesiumdecanat oder Talk die Gleitfähigkeit des Puders gesteigert werden.

Für die Akzeptanz eines Puders sind neben den abdeckenden und aufsaugenden Eigenschaften ferner die taktilen Eigenschaften von großer Bedeutung. Dementsprechend spielt auch die Korngröße der verwendeten Feststoffe eine entscheidende Rolle, da zu grobe Pigmente ein "Bremsgefühl" bewirken und die Haut stumpf machen.

Dekorative Puderformulierungen enthalten darüberhinaus verschiedenste Farbpigmente. Die Auswahl der Farbstoffe erfolgt in erster Linie mit dem Ziel, einen modisch aktuellen Farbton zu erzielen. Dabei muß allerdings beachtet werden, daß alle Farbstoffe und Pigmente hinsichtlich ihrer Verwendung gesetzlichen Bestimmungen unterliegen, in Deutschland beispielsweise der Verordnung über kosmetische Mittel.

Für Make-up-Formulierungen sind Pudersteine oder Kompaktpuder bzw. "Pancake-Make-up" wichtige Anwendungsformen geworden. Sie entsprechen von der Grundlage her losen Pudern, werden aber unter Zuhilfenahme eines Binders — wie z. B. Magnesiumstearat - unter Druck (etwa 40 bar) in flache Formen oder Näpfchen gepreßt. Kompaktpuder können auch hergestellt werden, indem man die Grundstoffe mit einer kleinen Menge Wasser und Binder zu einem Teig verarbeitet, der in kleine Formen gegossen wird, die man dann trocknen läßt, so daß der Inhalt fest wird. Auf diese Weise erhaltene Kompaktpuder finden vor allem Anwendung als Mascara, wobei zum Auftragen ein feuchter Pinsel verwendet wird.

Allen dekorativen Kosmetika ist gemeinsam, daß sie eine mehr oder weniger große Menge an Farbstoffen oder Farbpigmenten enthalten. Auch die Kombination löslicher Farbstoffe mit unlöslichen Pigmenten wird verwendet.

Von einem Deodorantpuder bzw. einem Antitranspirantpuder hingegen wird über die Wirksamkeit hinaus insbesondere erwartet, daß es bei der Anwendung in der Achseln keinen fettigen Eindruck erzeugt.

Der Stand der Technik hat eine Reihe von Nachteilen. Dazu zählt die Tatsache, daß Puderformulierungen üblicherweise kein Wasser enthalten können, da ansonsten die Pudermasse (unästhetisch) verkleben würde. Zwar können in Puderformulierungen auch flüssige Lipide eingearbeitet werden - beispielsweise um eine cremigere Textur zu erreichen — da aber wasserlösliche Wirkstoffe in der Regel nicht gut genug fettlöslich sind, lassen sie sich nicht in nennenswertem Maß in kosmetische Puder einbauen. Ein gewisser Wassergehalt in einer Puderformulierung wäre aber durchaus vorteilhaft, z. B. um beim Auftragen ein Frischegefühl auf der Haut zu erzeugen oder auch um die Kompatibilität des Puders mit der menschlichen Haut zu erhöhen. Weil aber Wasser die pulverige Erscheinungsform eines Puders zerstört, sind Puderformulierungen mit einem nennenswerten Wassergehalt nach dem Stand der Technik nicht machbar.

Eine Aufgabe der vorliegenden Erfindung war es, diesen Mängeln Abhilfe zu schaffen.

Eine weitere Aufgabe der vorliegenden Erfindung war es, Zubereitungen zu entwickeln, welche als Grundlage für kosmetische Desodorantien bzw. Antitranspirantien geeignet sind und die Nachteile des Standes der Technik nicht aufweisen und welche sich weiterhin durch gute Hautverträglichkeit auszeichnen.

Eine weitere Aufgabe der vorliegenden Erfindung war es, Puderformulierungen für die Anwendung als dekorative Kosmetika zu entwickeln, welche sich durch einen erhöhten Wassergehalt auszeichnen.

Erstaunlicherweise werden alle diese Aufgaben gelöst durch
kosmetische oder dermatologische Puderzubereitungen, enthaltend

1. Wasser,
2. mindestens ein Hydrokolloid und
3. mindestens einen hydrophoben oder hydrophob modifizierten partikulären Stoff sowie

gegebenenfalls weitere kosmetische oder pharmazeutische Hilfs-, Zusatz- und/oder Wirkstoffe enthaltend.

Die erfindungsgemäßen Zubereitungen sind in jeglicher Hinsicht überaus befriedigende Präparate, die erstaunlicherweise einen hohen Wassergehalt aufweisen können und dabei dennoch eine pulverige Konsistenz aufweisen. Sie zeichnen sich durch exzellente kosmetische Eigenschaften aus, sind rieselfähig und z. B. mit einem Pinsel auftragbar und bieten sich daher für den Einsatz in vielen Bereichen der pflegenden und dekorativen Kosmetik an. Die erfindungsgemäßen Zubereitungen sind ferner hervorragende Vehikel für verschiedenste Wirkstoffe.

Der Wasserphasenanteil der erfindungsgemäßen Formulierungen wird vorzugsweise aus dem Bereich von 5 bis 90 Gew.-% gewählt, besonders vorteilhaft aus dem Bereich 10 bis 80 Gew.-%, insbesondere 15 bis 75 Gew.-%, jeweils bezogen auf das Gesamtgewicht der Formulierungen.

"Hydrokolloid" ist die technologische Kurzbezeichnung für die an sich richtigere Bezeichnung "hydrophiles Kolloid". Hydrokolloide sind Makromoleküle, die eine weitgehend lineare Gestalt haben und über intermolekulare Wechselwirkungskräfte verfügen, die Neben- und Hauptvalenzbindungen zwischen den einzelnen Molekülen und damit die Ausbildung eines netzartigen Gebildes ermöglichen. Sie sind teilweise wasserlösliche natürliche oder synthetische Polymere, die in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie gleichzeitig die Beweglichkeit des Wassers einschränken.

Die Gruppe der Hydrokolloide läßt sich wie folgt einteilen in:

- organische, natürliche Verbindungen, wie beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine, Casein,
- organische, abgewandelte Naturstoffe, wie z. B. Carboxymethylcellulose und andere Celluloseether, Hydroxyethyl- und -propylcellulose und dergleichen,
- organische, vollsynthetische Verbindungen, wie z. B. Polyacryl- und Polymethacryl-Verbindungen, Vinylpolymere, Polycarbonsäuren, Polyether, Polyimine, Polyamide,
- anorganische Verbindungen, wie z. B. Polykieselsäuren, Tonmineralien wie Montmorillonite, Zeolithe, Kieselsäuren.

Erfindungsgemäß bevorzugte Hydrokolloide sind beispielsweise Methylcellulosen, als welche die Methylether der Cellulose bezeichnet werden. Sie zeichnen sich durch die folgende Strukturformel aus

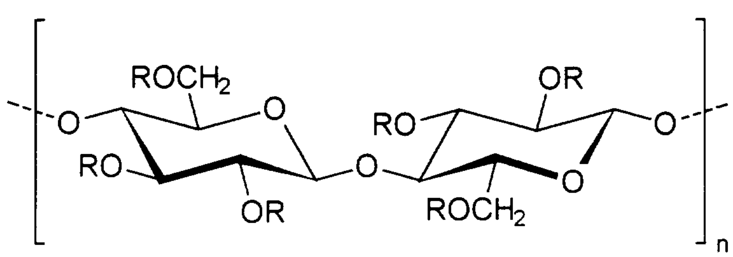

**Strukturformel I**

in der R ein Wasserstoff oder eine Methylgruppe darstellen kann.

Insbesondere vorteilhaft im Sinne der vorliegenden Erfindung sind die im allgemeinen ebenfalls als Methylcellulosen bezeichneten Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxypropyl)methylcellulosen, beispielsweise die unter der Handelsbezeichnung Methocel E4M bei der Dow Chemical Comp. erhältlichen.

Erfindungsgemäß ferner vorteilhaft ist Natriumcarboxymethylcellulose, das Natrium-Salz des Glykolsäureethers der Cellulose, für welches R in Strukturformel I ein Wasserstoff und/oder $CH_2$-COONa darstellen kann. Besonders bevorzugt ist die unter der Handelsbezeichnung Natrosol Plus 330 CS bei Aqualon erhältliche, auch als Cellulose Gum bezeichnete Natriumcarboxymethylcellulose.

Bevorzugt im Sinne der vorliegenden Erfindung ist ferner Xanthan (CAS-Nr. 11138-66-2), auch Xanthan Gummi genannt, welches ein anionisches Heteropolysaccharid ist, das in der Regel durch Fermentation aus Maiszucker gebildet und als Kaliumsalz isoliert wird. Es wird von Xanthomonas campestris und einigen anderen Species unter aeroben Bedingungen mit einem Molekulargewicht von $2\times10^6$ bis $24\times10^6$ produziert. Xanthan wird aus einer Kette mit β-1,4-gebundener Glucose (Cellulose) mit Seitenketten gebildet. Die Struktur der Untergruppen ("repeated units") besteht aus Glucose, Mannose, Glucuronsäure, Acetat und Pyruvat.

Erfindungsgemäß vorteilhafte Hydrokolloide sind ferner Polymere der Acrylsäure, insbesondere solche, die aus der Gruppe der sogenannten Carbomere oder Carbopole (Carbopol® ist eigentlich eine eingetragene Marke der B. F. Goodrich Company) gewählt werden. Carbopole sind Verbindungen der allgemeinen Strukturformel

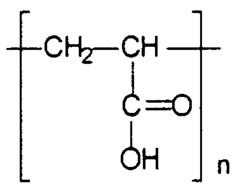
,

deren Molgewicht zwischen ca. 400 000 und mehr als 4 000 000 betragen kann. In die Gruppe der Carbopole gehören ferner Acrylat-Alkylacrylat-Copolymere, beispielsweise solche, die sich durch die folgende Struktur auszeichnen:

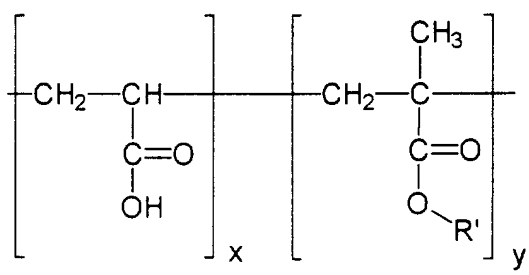

Darin stellen R' einen langkettigen Alkylrest und x und y Zahlen dar, welche den jeweiligen stöchiometrischen Anteil der jeweiligen Comonomere symbolisieren. Auch diese Carbopole sind vorteilhaft im Sinne der vorliegenden Erfindung.

Vorteilhafte Carbopole sind beispielsweise die Typen 907, 910, 934, 940, 941, 951, 954, 980, 981, 1342, 1382, 2984 und 5984, wobei diese Verbindungen einzeln oder in beliebigen Kombinationen untereinander vorliegen können. Besonders bevorzugt sind Carbopol 981, 1382 und 5984 (sowohl einzeln als auch in Kombination mit weiteren Hydrokolloiden).

Ferner vorteilhaft im Sinne der vorliegenden Erfindung sind die den Acrylat-Alkylacrylat-Copolymeren vergleichbaren Copolymere aus $C_{10\text{-}30}$-Alkylacrylaten und einem oder mehreren Monomeren der Acrylsäure, der Methacrylsäure oder deren Ester. Die INCI-Bezeichnung für solche Verbindungen ist "Acrylates/C 10-30 Alkyl Acrylate Crosspolymer". Insbesondere vorteilhaft sind die unter den Handelsbezeichnungen Pemulen TR1 und Pemulen TR2 bei der B. F. Goodrich Company erhältlichen.

Vorteilhafte Hydrokolloide im Sinne der vorliegenden Erfindung sind ferner Schichtsilikate. Silikate sind Salze und Ester (Kieselsäureester) der Orthokieselsäure $[Si(OH)_4]$ und deren Kondensationsprodukte. Die Silikate sind nicht nur die artenreichste Klasse der Mineralien, sondern auch geologisch und technisch außerordentlich wichtig. Über 80 % der Erdkruste bestehen aus Silikaten. Schichtsilikate (Phyllosilikate, Blattsilikate) sind (idealerweise) Silikat-Strukturen mit zweidimensional unendlichen Schichten aus $[SiO_4]^{4-}$-Tetraedern, wobei jedes Tetraeder über 3 Brücken-Sauerstoffe mit Nachbar-Tetraedern verbunden ist.

Chemische Formeln lassen sich für Schichtsilikate nur angenähert aufstellen, da sie ein großes Ionenaustausch-Vermögen besitzen und Silizium gegen Aluminium und dieses wiederum gegen Magnesium, $Fe^{2+}$, $Fe^{3+}$, Zn und dergleichen ausgetauscht werden kann. Die daraus möglicherweise resultierende negative Ladung der Schichten wird in der Regel durch Kationen, insbesondere durch $Na^+$ und $Ca^{2+}$ in Zwischenschicht-Positionen ausgeglichen.

Schichtsilikate können durch reversible Einlagerung von Wasser (in der 2- bis 7-fachen Menge) und anderen Pigmenten wie z. B. Alkoholen, Glykolen und dergleichen mehr aufquellen. Ihre Verwendung als Verdickungsmittel in kosmetischen Mitteln ist dementsprechend an sich bekannt. Allerdings konnte der Stand der Technik nicht den Weg zur vorliegenden Erfindung weisen.

Vorteilhafte Schichtsilikate im Sinne der vorliegenden Erfindung sind beispielsweise solche, deren größte Ausdehnungsrichtung im unmodifizierten und ungequollenen Zustand im Mittel eine Länge von weniger als 10 um hat. Beispielsweise können die mittleren Ausdehnungen der verwendeten modifizierten Schichtsilikatpartikel bei 1000 nm x 100 nm x 1 nm und darunter liegen. Die effektive Größe der modifizierten Schichtsilikatpartikel in einer kosmetischen oder dermatologischen Formulierung hängt selbstverständlich von der Menge an eingelagerten Pigmenten ab.

Vorteilhafte modifizierte Schichtsilikate im Sinne der vorliegenden Erfindung sind beispielsweise modifizierte Smektite (Smectite).

Smektite sind stets sehr feinkörnige (meist < 2 mm), überwiegend als lamellenförmige, moosartige oder kugelförmige Aggregate vorkommende Dreischicht-Tonminerale (2:1-Schichtsilikate), in denen eine zentrale Schicht aus oktaedrisch koordinierten Kationen sandwichartig von 2 Schichten aus [ $(Si,Al)O_4$]-Tetraedern umgeben ist. Smektite werden idealisiert durch die folgende Strukturformel beschrieben, worin weiß ausgefüllte Kreise Silizium- und/oder Aluminiumatome, hellgrau ausgefüllte Kreise Sauerstoffatome, dunkelgrau ausgefüllte Kreise Wasserstoffatome und schwarz ausgefüllte Kreise Aluminium-, Magnesium-, Eisenatome und/oder weitere Austauschkationen darstellen:

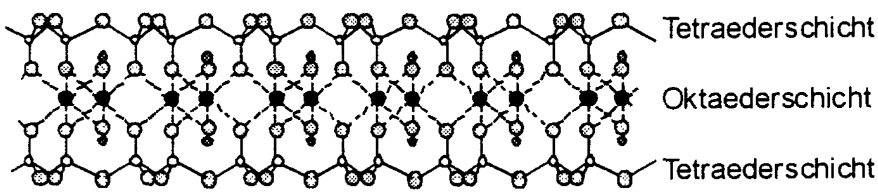


Vorteilhafte modifizierte Smektite sind z. B. modifizierte Montmorillonite. Montmorillonite werden durch die angenäherte chemische Formel $Al_2[(OH)_2/Si_4O_{10}] \cdot n\ H_2O$ bzw. $Al_2O_3 \cdot 4\ SiO_2 \cdot H_2O \cdot n\ H_2O$ beschrieben und stellen zu den dioktaedrischen Smektiten gehörende Tonmineralien dar.

Besonders vorteilhaft im Sinne der vorliegenden Erfindung sind ferner beispielsweise modifizierte Hektorite. Hektorite gehören zu den Smektiten und haben die angenäherte chemische Formel $M^+_{0,3}(Mg_{2,7}Li_{0,3})[Si_4O_{10}(OH)_2]$, worin $M^+$ meist $Na^+$ darstellt.

Vorteilhaft im Sinne der vorliegenden Erfindung sind ferner modifizierte Bentonite. Bentonite sind Tone und Gesteine, die Smektite, vor allem Montmorillonit, als Hauptminerale enthalten. Die "Roh"-Bentonite sind entweder Calcium-Bentonite (in Großbritannien als Fuller-Erden bezeichnet) oder Natrium-Bentonite (auch: Wyoming-Bentonite).

Modifizierte Schichtsilikate im Sinne der vorliegenden Erfindung sind Schichtsilikate, insbesondere die bereits genannten Schichtsilikattypen, deren Organophilie (auch: Lipophilie) — beispielsweise durch Umsetzung mit quarternären Ammonium-Verbindungen — erhöht wurde. Solche Schichtsilikate werden auch als organophile Schichtsilikate bezeichnet.

Besonders vorteilhaft im Sinne der vorliegenden Erfindung sind sogenannte Bentone, d. h. organische Derivate von Montmorilloniten (bzw. Bentoniten) und/oder Hectoriten, die durch Ionenaustausch-Reaktionen mit Alkylammonium-Basen hergestellt werden.

Vorteilhafte modifizierte Schichtsilikate im Sinne der vorliegenden Erfindung sind beispielsweise durch Umsetzung von Schichtsilikaten mit Quaternium-18 erhältlich. Quaternium-18 ist eine Mischung von quaternären Ammoniumchloridsalzen, welche durch die folgende Strukturformel beschrieben werden:

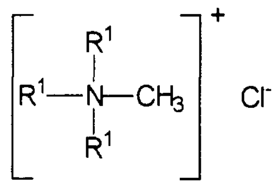

worin
die Reste $R^1$ unabhängig voneinander gewählt werden aus der Gruppe Methyl und hydrierte Talgreste mit einer Kettenlänge von 12 bis 20 Kohlenstoffatomen.

Erfindungsgemäß besonders bevorzugt sind Stearalkoniumhektorit, ein Reaktionsprodukt aus Hektorit und Stearalkoniumchlorid (Benzyldimethylstearylammoniumchlorid), und Quaternium-18 Hektorit, ein Reaktionsprodukt aus Hektorit und Quaternium-18, welche z. B. unter den Handelsbezeichnungen Bentone 27 und Bentone 38 bei Nordmann & Rassmann erhältlich sind.

Die Gesamtmenge an einem oder mehreren Hydrokolloiden wird in den fertigen kosmetischen oder dermatologischen Puderformulierungen vorteilhaft kleiner als 10 Gew.-%, bevorzugt zwischen 0,05 und 5 Gew.-%, insbesondere zwischen 0,1 und 3 Gew.-%, jeweils bezogen auf das Gesamtgewicht der Zubereitungen gewählt.

Die hydrophoben oder hydrophob modifizierten partikulären Stoffe im Sinne der vorliegenden Erfindung werden vorteilhaft aus der Gruppe der hochdispersen amorphen Siliziumdioxide gewählt.

Geeignete Siliciumdioxidpartikel sind beispielsweise sphärische Polyalkylsilsesquioxan-Partikel wie sie in der Europäischen Offenlegungsschrift 0 686 391 erwähnt werden. Solche Polyalkylsilsesquioxan-Partikel sind beispielsweise unter den Handelsbezeichnungen Aerosil R972, Aerosil 200V oder Aerosil R 812 bei der Firma Degussa erhältlich. Weitere erfindungsgemäße Siliziumdioxidpartikel sind unter den Handelsnamen Cab-o-Sil TS 610 oder Cab-o-Sil TS 530 von der Firme Cabot oder unter dem Namen Wacker HDK H2000 (Wacker) oder als Silica bead SBNP-43, SNBP-810 oder SB-705 von der Firma Miyoshi erhältlich.

Weitere vorteilhafte hydrophobe oder hydrophob modifizierte partikuläre Stoffe im Sinne der vorliegenden Erfindung sind mikrofeine Polymerpartikel, welche in der Zubereitung in Form von Feststoffen vorliegen. Erfindungsgemäß bevorzugt sind beispielsweise Polycarbonate, Polyether, Polyethylen, Polypropylen, Polyvinylchlorid, Polystyrol, Polyamide, Polyurethane, Polyacrylate und dergleichen mehr. Besonders vorteilhaft ist z. B. die Substanz mit der INCI-Bezeichnung *HDI / Trimethylol Hexyllactone Crosspolymer,* welche unter der Bezeichnung BPD-500/Plastic Powder D von der Firma Kobo erhältlich ist.

Erfindungsgemäß geeignet sind ferner beispielsweise mikrofeine Polyamid-Partikel, insbesondere die unter der Handelsbezeichnung SP-500 bei der Firma TORAY erhältlichen. Ferner vorteilhaft sind Polyamid 6- (auch: Nylon 6) bzw. Polyamid 12- (auch: Nylon 12) Partikel. Polyamid 6 ist das aus ε-Aminocapronsäure (6-Aminohexansäure) oder ε-Caprolactam aufgebaute Polyamid [Poly(ε-caprolactam)], und Polyamid 12 ist ein Poly(ε-laurinlactam) aus ε-Laurinlactam. Vorteilhaft im Sinne der vorliegenden Erfindung sind beispielsweise Orgasol® 1002 (Polyamid 6) und Orgasol® 2002 (Polyamid 12) von der Firma ELF ATOCHEM.

Weitere erfindungsgemäß vorteilhafte mikrofeine Polymerpartikel sind mikrofeine Polymethacrylate. Solche Partikel sind beispielsweise unter der Handelsbezeichnung POLYTRAP® bei der Firma DOW CHEMICAL erhältlich, aber auch unter den Namen BPA-500 (Kobo), Covabead LH 85 und Covabead PMMA (Wackherr), Ganzpearl GM-0600 (Presperse), Jurymer MB-1 (Nihon Junyaku), Microsphere M (Tomen), Microsphere M-100 (Matsumoto Yushi-Seiyaku) oder Micorpearl M 305, M 201 M 310 oder MHB (Seppic).

Der mittlere Partikeldurchmesser der verwendeten mikrofeinen Polymerpartikel wird vorzugsweise kleiner als 100 µm, besonders vorteilhaft kleiner als 50 µm gewählt. Dabei ist es im wesentlichen unerheblich, in welcher Form (Plättchen, Stäbchen, Kügelchen etc.) die verwendeten Polymerpartikel vorliegen.

Erfindungsgemäß vorteilhaft sind anorganische Pigmente, die oberflächlich wasserabweisend behandelt ("gecoatet") sind, wobei gleichzeitig ein hydrophober Charakter dieser Pigmente gebildet werden bzw. erhalten bleiben soll. Diese Oberflächenbehandlung kann darin bestehen, daß die Pigmente nach an sich bekannten Verfahren mit einer dünnen hydrophoben Schicht versehen werden.

Ein solches Verfahren, das im folgenden am Beispiel von Titandioxid beschrieben wird, besteht beispielsweise darin, daß die hydrophobe Oberflächenschicht nach einer Reaktion gemäß

$$n\ TiO_2 + m\ (RO)_3\ Si\text{-}R' \rightarrow n\ TiO_2\ (\text{oberfl.})$$

erzeugt wird. n und m sind dabei nach Belieben einzusetzende stöchiometrische Parameter, R und R' die gewünschten

organischen Reste. Besonders vorteilhaft sind $TiO_2$-Pigmente, beispielsweise die mit Aluminiumstearat beschichteten, unter der Handelsbezeichnung MT 100 T bei der Firma TAYCA erhältlichen.

Eine weitere vorteilhafte Beschichtung besteht aus Dimethylpolysiloxan (auch: Dimethicone), einem Gemisch vollmethylierter, linearer Siloxanpolymere, die endständig mit Trimethylsiloxy-Einheiten blockiert sind. Vorteilhaft im Sinne der vorliegenden Erfindung sind auch Zinkoxid-Pigmente, die auf diese Weise beschichtet werden.

Vorteilhaft ist ferner, wenn die hydrophoben anorganischen Pigmente mit einem Gemisch aus Dimethylpolysiloxan, insbesondere Dimethylpolysiloxan mit einer durchschnittlichen Kettenlänge von 200 bis 350 Dimethylsiloxan-Einheiten, und Silicagel beschichtet sind, welches auch als Simethicone bezeichnet wird. Es ist insbesondere von Vorteil, wenn die anorganischen Pigmente zusätzlich mit Aluminiumhydroxid bzw. Aluminiumoxidhydrat (auch: Alumina, CAS-Nr.: 1333-84-2) beschichtet sind. Besonders vorteilhaft sind Titandioxide, die mit Simethicone und Alumina beschichtet sind, wobei die Beschichtung auch Wasser enthalten kann. Ein Beispiel hierfür ist das unter dem Handelsnamen Eusolex T2000 bei der Firma Merck erhältliche Titandioxid oder z.B. die Pigmente der ST-PEG/MOD Serie von der Firma Miyoshi.

Vorteilhaft im Sinne der vorliegenden Erfindung sind ferner Mischungen verschiedener anorganischer, hydrophober Pigmenttypen sowohl innerhalb eines Kristalls, beispielsweise als Eisenmischoxid oder Talkum (Magnesiumsilicat), als auch durch Mischung mehrerer Metalloxidtypen innerhalb einer Zubereitung. Besonders vorteilhaft sind Magnesiumsilicate, beispielsweise die unter der Handelsbezeichnung Talkum Micron bei der Firma Grolmann erhältlichen.

Ein weiterer vorteilhafter hydrophober oder hydrophob modifizierter partikulärer Stoff im Sinne der vorliegenden Erfindung ist ferner Bornitrid, beispielsweise die im folgenden aufgelisteten Bornitride:

| **Handelsname** | **erhältlich bei:** |
|---|---|
| Boron Nitride Powder | Advanced Ceramics |
| Boron Nitride Powder | Sintec Keramik |
| Ceram Blanche | Kawasaki |
| HCST Boron Nitride | Stark |
| Très BN® | Carborundum |
| Wacker-Bomitrid BNP | Wacker-Chemie |

Vorteilhaft ist es, den mittleren Partikeldurchmesser der verwendeten Bornitridpartikel kleiner als 20 µm, besonders vorteilhaft kleiner als 15 µm zu wählen.

Ebenfalls vorteilhaft sind Bornitridpartikel, die oberflächlich wasserabweisend behandelt ("gecoatet") sind, wobei gleichzeitig der hydrophobe Charakter gebildet werden bzw. erhalten bleiben soll.

Eine vorteilhafte Beschichtung der Bornitridpartikel besteht aus Dimethylpolysiloxan (auch: Dimethicone), einem Gemisch vollmethylierter, linearer Siloxanpolymere, die endständig mit Trimethylsiloxy-Einheiten blockiert sind. Vorteilhaft sind beispielsweise die bei der Firma Carborundum unter der Handelsbezeichnung Très BN® UHP 1106 erhältlichen, mit Dimethicone behandelten Bornitridpartikel.

Vorteilhaft ist ferner eine Beschichtung der Bornitridpartikel mit Polymethylhydrogensiloxan, einem linearen Polysiloxan, welches auch als Methicone bezeichnet wird. Vorteilhafte, mit Methicone behandelte Bornitridpartikel sind beispielsweise die bei der Firma Carborundum unter der Handelsbezeichnung Très BN® UHP 1107 erhältlichen.

Die hydrophoben oder hydrophob modifizierten partikulären Stoffe im Sinne der vorliegenden Erfindung werden ferner vorzugsweise gewählt aus der Gruppe der hydrophob modifizierten Polysaccharide, welche keine verdikkenden Eigenschaften zeigen.

Solche hydrophoben Polysaccharide sind beispielsweise durch Umsetzung von Stärke mit mono-, bi- oder polyfunktionellen Reagenzien bzw. Oxidations-Mitteln in weitgehend polymeranalog verlaufenden Reaktionen erhältlich.

Diese Reaktionen basieren im wesentlichen auf Umwandlungen der Hydroxy-Gruppen der Polyglucane durch Veretherung, Veresterung oder selektive Oxidation. Dabei entstehen z. B. sogenannte Stärkeether und Stärkeester der allgemeinen Strukturformel

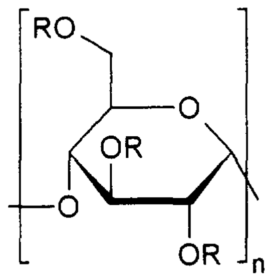

worin R beispielsweise ein Wasserstoff und/oder einen Alkyl- und/oder Aralkylrest (im Fall der Stärkeether) oder ein Wasserstoff und/oder einen organischen und/oder anorganischen Säure-Rest (im Fall der Stärkeester) darstellen kann. Stärkeether und Stärkeester sind vorteilhaft im Sinne der vorliegenden Erfindung.

Besonders vorteilhaft sind Stärkeether, z. B. solche, die durch Veretherung von Stärke mit Tetramethylolacetylendiharnstoff erhältlich sind und welche als Amylum non mucilaginosum (nicht quellende Stärke) bezeichnet werden.

Insbesondere vorteilhaft sind ferner Stärkeester und/oder deren Salze, beispielsweise die Natrium- und/oder Aluminiumsalze niedrigsubstituierter Halbester der Stärke, insbesondere Natrium Stärke n-Octenylsuccinat der Strukturformel (I), worin R sich durch die folgende Struktur auszeichnet

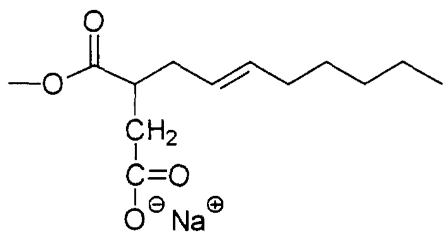

und welches z. B. unter der Handelsbezeichnung Amiogum® 23 bei der Firma CERE-STAR erhältlich ist, sowie Aluminium Stärke Octenylsuccinat, insbesondere die unter den Handelsbezeichnungen Dry Flo® Elite LL und Dry Flo® PC bei der Firma National Starch & Chemical erhältlichen.

Vorteilhaft ist es, den mittleren Partikeldurchmesser der verwendeten, modifizierten Polysaccharide kleiner als 20 µm, besonders vorteilhaft kleiner als 15 µm zu wählen.

Die Liste der genannten modifizierten Polysaccharide, die hydrophobe Pigmente im Sinne der vorliegenden Erfindung darstellen können, soll selbstverständlich nicht limitierend sein. Modifizierte Polysaccharide, die erfindungsgemäße hydrophobe Pigmente darstellen, sind auf zahlreichen, an sich bekannten Wegen, sowohl chemischer als auch physikalischer Natur erhältlich. Zur Herstellung solcher Polysaccharide sind auch neue Wege prinzipiell denkbar. Wesentlich dabei ist, daß die modifizierten Polysaccharide hydrophobe Eigenschaften zeigen und daß sie nicht verdickend wirken.

Weitere erfindungsgemäß vorteilhafte hydrophobe Substanzen sind z. B. Silikonelastomeren, Silikongummis oder Silikonharze.

So kann es z. B. von erheblichem Vorteil sein, solche Silikonelastomeren in Zubereitungen gemäß der vorliegenden Erfindung zu verwenden, wie sie beispielsweise in den US-Patenten US 4980167 oder US 4742142 beschrieben werden. Vorteilhafte Silikonelastomeren sind z. B. solche, welche unter den Namen KSG6 von Shin Etsu, Tefil E-505C oder Trefil E-506C von Dow Corning, Gransil von Grant Industries (SR-CYC, SR-DMF10, SR-DC556) vertrieben werden sowie solche, die in Form von vorgefertigten Gelen verkauft werden (wie z. B. KSG15, KSG17, KSG16, KSG18 von Shin Etsu, Gransil SP 5CYC Gel, Gransil SR DMF 10 Gel, Gransil SR DC 556 Gel, Gransil GCM, Gransil PM Gel, Gransil DMG-5, SF 1204 und JK 113 von Gereral Electric).

Weitere vorteilhafte Silikonelastomeren können gewählt werden aus der Gruppe der Vinyl Dimethicone Crosspolymere, wie z. B. das Dow Corning 9506 Cosmetic Powder von Dow Corning (INCI: Dimethicone / Vinyl Dimethicone Crosspolymer).

Ferner vorteilhaft sind Polymethylsilsesquioxane, beispielsweise die unter den Handelsnamen Tospearl 2000 B von GE Bayer Silikones, Tospearl 145A von Toshiba, AEC Silicone Resin Spheres von A & E Connock oder Wacker - Belsil PMS MK von der Wacker-Chemie angebotenen.

Vorteilhaft in allen vorgenannten Fällen ist es, die Gesamtkonzentration aller hydrophoben oder hydrophob modifizierten partikulären Stoffe (eine oder mehrere Verbindungen) größer als 0,05 Gew.-%, besonders vorteilhaft zwischen 0,5 Gew.-% und 50 Gew.-%, insbesondere zwischen 2 und 25 Gew.-% zu wählen, jeweils bezogen auf das

Gesamtgewicht der Zubereitungen.

Die erfindungsgemäßen Puderzubereitungen können ferner vorteilhaft auch sogenannte Metallseifen (d. h. die Salze höherer Fettsäuren mit Ausnahme der Alkalisalze) enthalten, wie beispielsweise Aluminium-Stearat, Zink-Stearat und/oder Magnesium-Stearat.

Die erfindungsgemäßen Puderformulierungen können wie üblich zusammengesetzt sein und beispielsweise zur Behandlung und Pflege der Haut, als Deoprodukt und als Schminkprodukt in der dekorativen Kosmetik oder als Lichtschutzpräparat dienen. Es ist gegebenenfalls möglich und vorteilhaft, die erfindungsgemäßen Zusammensetzungen als Grundlage für pharmazeutische Formulierungen zu verwenden.

Zur Anwendung werden die erfindungsgemäßen kosmetischen und dermatologischen Zubereitungen in der für Kosmetika üblichen Weise auf die Haut in ausreichender Menge aufgebracht.

Die erfindungsgemäßen kosmetischen und dermatologischen Zubereitungen können Farbstoffe und/oder Farbpigmente enthalten, insbesondere wenn sie in Form von dekorativen Pudern vorliegen. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. $Fe_2O_3$, $Fe_3O_4$, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem *Rowe Colour Index, 3. Auflage, Society of Dyers and Colourists, Bradford, England, 1971* entnommen.

| Chemische oder sonstige Bezeichnung | CIN | Farbe |
|---|---|---|
| Pigment Green | 10006 | grün |
| Acid Green 1 | 10020 | grün |
| 2,4-Dinitrohydroxynaphthalin-7-sulfosäure | 10316 | gelb |
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro-1'-phenylazo)-2-hydroxynaphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |
| Pigment Brown 1 | 12480 | braun |
| 4-(2'-Methoxy-5'-sulfosäurediethylamid-1'-phenylazo)-3-hydroxy-5"-chloro-2",4"-dimethoxy-2-naphthoesäureanilid | 12490 | rot |
| Disperse Yellow 16 | 12700 | gelb |
| 1-(4-Sulfo-1-phenylazo)-4-amino-benzol-5-sulfosäure | 13015 | gelb |
| 2,4-Dihydroxy-azobenzol-4'-sulfosäure | 14270 | orange |
| 2-(2,4-Dimethylphenylazo-5-sulfosäure)-1-hydroxynaphthalin-4-sulfosäure | 14700 | rot |
| 2-(4-Sulfo-1-naphthylazo)-1-naphthol-4-sulfosäure | 14720 | rot |
| 2-(6-Sulfo-2,4-xylylazo)-1-naphthol-5-sulfosäure | 14815 | rot |
| 1-(4'-Sulfophenylazo)-2-hydroxynaphthalin | 15510 | orange |
| 1-(2-Sulfosäure-4-chlor-5-carbonsäure-1-phenylazo)-2-hydroxynaphthalin | 15525 | rot |
| 1-(3-Methyl-phenylazo-4-sulfosäure)-2-hydroxynaphthalin | 15580 | rot |
| 1-(4',(8')-Sulfosäurenaphthylazo)-2-hydroxynaphthalin | 15620 | rot |
| 2-Hydroxy-1,2'-azonaphthalin-1'-sulfosäure | 15630 | rot |
| 3-Hydroxy-4-phenylazo-2-naphthylcarbonsäure | 15800 | rot |
| 1-(2-Sulfo-4-methyl-1-phenylazo)-2-naphthylcarbonsäure | 15850 | rot |
| 1-(2-Sulfo-4-methyl-5-chlor-1-phenylazo)-2-hydroxy-naphthalin-3-carbonsäure | 15865 | rot |
| 1-(2-Sulfo-1-naphthylazo)-2-hydroxynaphthalin-3-carbonsäure | 15880 | rot |
| 1-(3-Sulfo-1-phenylazo)-2-naphthol-6-sulfosäure | 15980 | orange |
| 1-(4-Sulfo-1-phenylazo)-2-naphthol-6-sulfosäure | 15985 | gelb |

(fortgesetzt)

| Chemische oder sonstige Bezeichnung | CIN | Farbe |
|---|---|---|
| Allura Red | 16035 | rot |
| 1-(4-Sulfo-1-naphthylazo)-2-naphthol-3,6-disulfosäure | 16185 | rot |
| Acid Orange 10 | 16230 | orange |
| 1-(4-Sulfo-1-naphthylazo)-2-naphthol-6,8-disulfosäure | 16255 | rot |
| 1-(4-Sulfo-1-naphthylazo)-2-naphthol-3,6,8-trisulfosäure | 16290 | rot |
| 8-Amino-2 -phenylazo- 1 -naphthol-3,6-disulfosäure | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Sulfo-1-phenylazo)-1-(4-sulfophenyl)-5-hydroxy-pyrazolon-3-carbonsäure | 19140 | gelb |
| Pigment Yellow 16 | 20040 | gelb |
| 2,6-(4'-Sulfo-2", 4"-dimethyl)-bis-phenylazo)1,3-dihydroxybenzol | 20170 | orange |
| Acid Black 1 | 20470 | schwarz |
| Pigment Yellow 13 | 21100 | gelb |
| Pigment Yellow 83 | 21108 | gelb |
| Solvent Yellow | 21230 | gelb |
| Acid Red 163 | 24790 | rot |
| Acid Red 73 | 27290 | rot |
| 2-[4'-(4"-Sulfo-1"-phenylazo)-7'-sulfo-1'-naphthylazo]-1-hydroxy-7-aminonaphthalin-3,6-disulfosäure | 27755 | schwarz |
| 4'-[(4"-Sulfo-1"-phenylazo)-7'-sulfo-1'-naphthylazo]-1-hydroxy-8-acetyl-aminonaphthalin-3,5-disulfosäure | 28440 | schwarz |
| Direct Orange 34, 39, 44, 46, 60 | 40215 | orange |
| Food Yellow | 40800 | orange |
| trans-β-Apo-8'-Carotinaldehyd ($C_{30}$) | 40820 | orange |
| trans-Apo-8'-Carotinsäure ($C_{30}$)-ethylester | 40825 | orange |
| Canthaxanthin | 40850 | orange |
| Acid Blue 1 | 42045 | blau |
| 24-Disulfo-5-hydroxy-4'-4"-bis-(diethylamino)triphenyl-carbinol | 42051 | blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | grün |
| Acid Blue 7 | 42080 | blau |
| (N-Ethyl-p-sulfobenzyl-amino)-phenyl-(2-sulfophenyl)-methylen-(N-ethyl-N-p-sulfo-benzyl) $\Delta^{2,5}$-cyclohexadienimin | 42090 | blau |
| Acid Green 9 | 42100 | grün |
| Diethyl-di-sulfobenzyl-di-4-amino-2-chlor-di-2-methyl-fuchsonimmonium | 42170 | grün |
| Basic Violet 14 | 42510 | violett |
| Basic Violet 2 | 42520 | violett |
| 2'-Methyl-4'-(N-ethyl-N-m-sulfobenzyl)-amino-4"-(N-diethyl)-amino-2-methyl-N-ethylN-m-sulfobenzyl-fuchsonimmonium | 42735 | blau |
| 4'-(N-Dimethyl)-amino-4"-(N-phenyl)-aminonaphtho-N-dimethylfuchsonimmonium | 44045 | blau |
| 2-Hydroxy-3,6-disulfo-4,4'-bis-dimethylaminonaphthofuchsonimmonium | 44090 | grün |
| Acid Red 52 | 45100 | rot |
| 3-(2'-Methylphenylamino)-6-(2'-methyl-4'-sulfophenylamino)-9-(2"-carboxyphenyl)-xantheniumsalz | 45190 | violett |
| Acid Red 50 | 45220 | rot |
| Phenyl-2-oxyfluoron-2-carbonsäure | 45350 | gelb |

(fortgesetzt)

| Chemische oder sonstige Bezeichnung | CIN | Farbe |
|---|---|---|
| 4,5-Dibromfluorescein | 45370 | orange |
| 2,4,5,7-Tetrabromfluorescein | 45380 | rot |
| Solvent Dye | 45396 | orange |
| Acid Red 98 | 45405 | rot |
| 3',4',5',6'-Tetrachlor-2,4,5,7-tetrabromfluorescein | 45410 | rot |
| 4,5-Diiodfluorescein | 45425 | rot |
| 2,4,5,7-Tetraiodfluorescein | 45430 | rot |
| Chinophthalon | 47000 | gelb |
| Chinophthalon-disulfosäure | 47005 | gelb |
| Acid Violet 50 | 50325 | violett |
| Acid Black 2 | 50420 | schwarz |
| Pigment Violet 23 | 51319 | violett |
| 1,2-Dioxyanthrachinon, Calcium-Aluminiumkomplex | 58000 | rot |
| 3-Oxypyren-5,8,10-sulfosäure | 59040 | grün |
| 1-Hydroxy-4-N-phenyl-aminoanthrachinon | 60724 | violett |
| 1-Hydroxy-4-(4'-methylphenylamino)-anthrachinon | 60725 | violett |
| Acid Violet 23 | 60730 | violett |
| 1,4-Di(4'-methyl-phenylamino)-anthrachinon | 61565 | grün |
| 1,4-Bis-(o-sulfo-p-toluidino)-anthrachinon | 61570 | grün |
| Acid Blue 80 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfosäure | 73015 | blau |
| 4,4'-Dimethyl-6,6'-dichlorthioindigo | 73360 | rot |
| 5,5'-Dichlor-7,7'-dimethylthioindigo | 73385 | violett |
| Quinacridone Violet 19 | 73900 | violett |
| Pigment Red 122 | 73915 | rot |
| Pigment Blue 16 | 74100 | blau |
| Phthalocyanine | 74160 | blau |
| Direct Blue 86 | 74180 | blau |
| Chlorierte Phthalocyanine | 74260 | grün |
| Natural Yellow 6,19; Natural Red 1 | 75100 | gelb |
| Bixin, Nor-Bixin | 75120 | orange |
| Lycopin | 75125 | gelb |
| trans-alpha-, beta- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na, Al, Ca) der Karminsäure | 75470 | rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| Wasserhaltige Aluminiumsilikate | 77004 | weiß |
| Ultramarin | 77007 | blau |
| Pigment Red 101 und 102 | 77015 | rot |
| Bariumsulfat | 77120 | weiß |

(fortgesetzt)

| Chemische oder sonstige Bezeichnung | CIN | Farbe |
|---|---|---|
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268:1 | schwarz |
| Chromoxid | 77288 | grün |
| Chromoxid, wasserhaltig | 77289 | grün |
| Pigment Blue 28, Pigment Green 14 | 77346 | grün |
| Pigment Metal 2 | 77400 | braun |
| Gold | 77480 | braun |
| Eisenoxide und -hydoxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)-hexacyanoferrat | 77510 | blau |
| Pigment White 18 | 77713 | weiß |
| Mangananimoniumdiphosphat | 77742 | violett |
| Manganphosphat; $Mn_3(PO_4)_2 \cdot 7\ H20$ | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyryliumsalze, Anthocyane | | rot |
| Aluminium-, Zink-, Magnesium- und Calciumstearat | | weiß |
| Bromthymolblau | | blau |
| Bromkresolgrün | | grün |
| Acid Red 195 | | rot |

Sofern die erfindungsgemäßen Puderformulierungen in Form von Gesichtspudern vorliegen, ist es günstig, als Farbstoff eine oder mehrer Substanzen aus der folgenden Gruppe zu wählen: 2,4-Dihydroxyazobenzol, 1-(2'-Chlor-4'-nitro-1'-phenylazo)-2-hydroxynaphthalin, Ceresrot, 2-(4-Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 4-(4-Sulfo-1-phenylazo)-1-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77 492), Manganammoniumdiphosphat und Titandioxid.

Ferner vorteilhaft sind öllösliche Naturfarbstoffe, wie z. B. Paprikaextrakte, β-Carotin oder Cochenille.

Vorteilhaft im Sinne der vorliegenden Erfindung sind ferner Puderformulierungen mit einem Gehalt an Perlglanzpigmenten. Bevorzugt sind insbesondere die im folgenden aufgelisteten Arten von Perlglanzpigmenten:

1. Natürliche Perlglanzpigmente, wie z. B.

- "Fischsilber" (Guanin/Hypoxanthin-Mischkristalle aus Fischschuppen) und

- "Perlmutt" (vermahlene Muschelschalen)

2. Monokristalline Perlglanzpigmente wie z. B. Bismuthoxychlorid (BiOCl)
3. Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteihaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| Gruppe | Belegung / Schichtdicke | Farbe |
|---|---|---|
| **Silberweiße Perlglanzpigmente** | $TiO_2$: 40-60 nm | silber |
| **Interferenzpigmente** | $TiO_2$: 60 - 80 nm | gelb |
| | $TiO_2$: 80 - 100 nm | rot |
| | $TiO_2$: 100 - 140 nm | blau |
| | $TiO_2$: 120 - 160 nm | grün |
| **Farbglanzpigmente** | $Fe_2O_3$ | bronze |
| | $Fe_2O_3$ | kupfer |
| | $Fe_2O_3$ | rot |
| | $Fe_2O_3$ | rotviolett |
| | $Fe_2O_3$ | rotgrün |
| | $Fe_2O_3$ | schwarz |
| **Kombinationspigmente** | $TiO_2$ / $Fe_2O_3$ | Goldtöne |
| | $TiO_2$/$Cr_2O_3$ | grün |
| | $TiO_2$/ Berliner Blau | tiefblau |
| | $TiO_2$ / Carmin | rot |

Besonders bevorzugt sind z.B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit $TiO_2$ und $Fe_2O_3$ beschichtete $SiO_2$-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Eisenperlglanzpigmente, welche ohne die Verwendung von Glimmer hergestellt werden. Solche Pigmente sind z. B. unter dem Handelsnamen Sicopearl Kupfer 1000 bei der Firma BASF erhältlich.

Besonders vorteilhaft sind ferner auch Effektpigmente, welche unter der Handelsbezeichnung Metasomes Standard / Glitter in verschiedenen Farben (yello, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (CI) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.-% bis 30 Gew.-%, vorzugsweise von 0,5 bis 15 Gew.-%, insbesondere von 1,0 bis 10 Gew.-% gewählt, jeweils bezogen auf das Gesamtgewicht der Zubereitungen.

Eine erstaunliche Eigenschaft der erfindungsgemäßen Zubereitungen ist, daß diese sehr gute Vehikel für kosmetische oder dermatologische Wirkstoffe in die Haut sind, wobei vorteilhafte Wirkstoffe Antioxidantien sind, welche die Haut vor oxidativer Beanspruchung schützen können.

Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden. Es ist dabei vorteilhaft, Antioxidantien als einzige Wirkstoffklasse zu verwenden, etwa dann, wenn eine kosmetische oder dermatologische Anwendung im Vordergrund steht, wie z. B. die Bekämpfung der oxidativen Beanspruchung der Haut. Es ist aber auch günstig, die erfindungsgemäßen Puderzubereitungen mit einem Gehalt an einem oder mehreren Antioxidantien zu versehen, wenn die Zubereitungen einem anderen Zweck dienen sollen, z. B. als Desodorantien oder Sonnenschutzmittel.

Besonders vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den erfindungsgemäßen Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Erfindungsgemäß können die Wirkstoffe (eine oder mehrere Verbindungen) auch sehr vorteilhaft gewählt werden aus der Gruppe der lipophilen Wirkstoffe, insbesondere aus folgender Gruppe:

Acetylsalicylsäure, Atropin, Azulen, Hydrocortison und dessen Derivaten, z.B. Hydrocortison-17-valerat, Vitamine, z.B. Ascorbinsäure und deren Derivate, Vitamine der B- und D-Reihe, sehr günstig das Vitamin $B_1$, das Vitamin $B_{12}$ das Vitamin $D_1$, aber auch Bisabolol, ungesättigte Fettsäuren, namentlich die essentiellen Fettsäuren (oft auch Vitamin F genannt), insbesondere die gamma-Linolensäure, Ölsäure, Eicosapentaënsäure, Docosahexaënsäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkernöl, Fischöle, Lebertran aber auch Ceramide und ceramidähnliche Verbindungen und so weiter.

Vorteilhaft ist es auch, die Wirkstoffe aus der Gruppe der rückfettenden Substanzen zu wählen, beispielsweise Purcellinöl, Eucerit® und Neoceht® .

Erfindungsgemäß können die Wirkstoffe (eine oder mehrere Verbindungen) - je nach dem, welche kosmetische oder dermatologische Anwendung im Vordergrund steht - auch sehr vorteilhaft gewählt werden aus der Gruppe der hydrophilen Wirkstoffe, insbesondere aus folgenden Gruppen:

- ♦ wasserlösliche Vitamine, wie z. B. Panthenol,
- ♦ Proteine und/oder Aminosäuren (Glycin, Alanin, Valin, Leucin, Isoleucin, Phenylalanin, Tyrosin, Prolin, Hydroxyprolin, Serin, Threonin, Cystein, Methionin, Tryptophan, Arginin und dergleichen),
- ♦ antitranspirante Wirkstoffe, wie beispielsweise Aluminiumsalze (Aluminiumchlorid, -nitrat, -sulfat, -acetat, Aluminiumchlorhydrat) und/oder die entsprechenden Zink-, Magnesium- und Zirkoniumverbindungen sowie Zirkonium/ Aluminium-Mischsalze und dergleichen mehr,
- ♦ Keratolytika (wie z. B. Harnstoff etc.),
- ♦ α-Hydroxycarbonsäuren, α-Ketocarbonsäuren und β-Hydroxycarbonsäuren und deren Salze, wie z. B. Salicylsäure, Äpfelsäure, Milchsäure, Citronensäure, Weinsäure, Brenztraubensäure usw.

Die Liste der genannten Wirkstoffe bzw. Wirkstoffkombinationen, die in den erfindungsgemäßen Puderzubereitungen verwendet werden können, soll selbstverständlich nicht limitierend sein.

Die Wasserphase der erfindungsgemäßen Puderzubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte sowie Elektrolyte und/oder Zuckerderivate.

Günstig sind auch solche kosmetischen und dermatologischen Zubereitungen, die in der Form eines Sonnenschutzmittels vorliegen. Vorzugsweise enthalten diese neben den erfindungsgemäßen Wirkstoffkombinationen zusätzlich mindestens eine UV-A-Filtersubstanz und/oder mindestens eine UV-B-Filtersubstanz. Solche Formulierungen können, obgleich nicht notwendig, gegebenenfalls auch ein oder mehrere anorganische Pigmente als UV-Filtersubstanzen enthalten.

Bevorzugt sind anorganische Pigmente auf Basis von Metalloxiden und/oder anderen in Wasser schwerlöslichen oder unlöslichen Metallverbindungen, insbesondere der Oxide des Titans ($TiO_2$), Zinks (ZnO), Eisens (z. B. $Fe_2O_3$), Zirkoniums ($ZrO_2$), Siliciums ($SiO_2$), Mangans (z. B. MnO), Aluminiums ($Al_2O_3$), Cers (z. B. $Ce_2O_3$), Mischoxiden der entsprechenden Metalle sowie Abmischungen aus solchen Oxiden.

Auch ein zusätzlicher Gehalt an stabilisierend wirkenden Titandioxid- und/oder Zinkoxidpartikeln kann selbstverständlich vorteilhaft sein, ist aber im Sinne der vorliegenden Erfindung nicht notwendig.

Es ist auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes gewöhnlich UV-A- bzw. UV-B-Filtersubstanzen eingearbeitet.

Auch stellen UV-Schutzsubstanzen, ebenso wie Antioxidantien und, gewünschtenfalls, Konservierungsstoffe, einen wirksamen Schutz der Zubereitungen selbst gegen Verderb dar.

Vorteilhaft enthalten erfindungsgemäße Zubereitungen Substanzen, die UV-Strahlung im UV-A- und/oder UV-B-Bereich absorbieren, wobei die Gesamtmenge der Filtersubstanzen z. B. 0,1 Gew.-% bis 30 Gew.-%, vorzugsweise 0,5 bis 20 Gew.-%, insbesondere 1,0 bis 15,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz sowie das 1,4-di(2-oxo-10-Sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate. Insbesondere bevorzugt sind das 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist, und das 4,4',4''-(1,3,5-Triazin-2,4,6-triyltriimino)-tris-benzoäsäure-tris(2-ethylhexylester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Besonders bevorzugte UV-Filtersubstanz im Sinne der vorliegenden Erfindung ist ferner ein unsymmetrisch substituiertes s-Triazin, welches auch als Dioctylbutylamidotriazon (INCI: Dioctylbutamidotriazone) bezeichnet wird und unter der Handelsbezeichnung UVASORB HEB bei Sigma 3V erhältlich ist.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-([4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2''-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2''-methyl-propyloxy)-2-hydroxy]-phenyl}-6-(4-methoxyphenyl)-1,3,5-triazin.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die UV-B-Filter können öllöslich oder wasserlöslich sein. Vorteilhafte öllösliche UV-B-Filtersubstanzen sind z. B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
- sowie an Polymere gebundene UV-Filter.

Vorteilhafte wasserlösliche UV-B-Filtersubstanzen sind z. B.:

- Salze der 2-Phenylbenzimidazol-5-sulfonsäure, wie ihr Natrium-, Kalium- oder ihr Triethanolammonium-Salz, sowie die Sulfonsäure selbst;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bomylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist.

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Ferner kann es gegebenenfalls von Vorteil sein, erfindungsgemäß weitere UV-A-und/oder UV-B-Filter in kosmetische oder dermatologische Zubereitungen einzuarbeiten, beispielsweise bestimmte Salicylsäurederivate wie 4-Isopropylbenzylsalicylat, 2-Ethylhexylsalicylat (= Octylsalicylat), Homomenthylsalicylat.

Die Liste der genannten UV-Filter, die im Sinne der vorliegenden Erfindung eingesetzt werden können, soll selbstverständlich nicht limitierend sein.

Erfindungsgemäße Puderformulierungen sind erhältlich, indem man ein Gel bestehend aus einem oder mehreren Hydrokolloiden und Wasser herstellt. Dieses Gel wird mit den gewünschten hydrophilen Wirkstoffen versetzt und dann mit einer Substanz oder einer Mischung aus Substanzen aus der Gruppe der hydrophoben oder hydrophob modifizierten Partikel versetzt. Zu dieser Masse werden dann noch Pigmente und Füllstoffe gegeben.

Derartige Gele können außer mit hydrophilen Wirkstoffen auch mit einer Menge an lipophilen Stoffen beladen werden. Die Menge der lipophilen Stoffe ist dabei vorteilhaft kleiner als 10 Gew.-%, besonders vorteilhaft kleiner 5 Gew.-% zu wählen, jeweils bezogen auf das Gesamtgewicht des Gels.

Gegenstand der Erfindung ist daher auch ein Verfahren zur Herstellung von Puderformulierungen, das dadurch gekennzeichnet ist, daß man in an sich bekannter Weise aus einem oder mehreren Hydrokolloiden ein Gel herstellt, gewünschtenfalls einen oder mehrere hydrophile Wirkstoffe sowie weitere kosmetische oder pharmazeutische Hilfs-, Zusatz- und/oder Wirkstoffe zugibt, und die Gelphase anschließend mit mindestens einer Sorte hydrophober oder hydrophob modifizierter Partikel versetzt und gewünschtenfalls weitere (färbende) Pigmente und/oder Füllstoffe hinzufügt.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

**Beispiele:**

**Beispiel 1 hautbefeuchtender Puder:**

| | Gew.-% |
|---|---|
| PEG-8 (Polyethylenglycol 400) | 5,00 |
| Ethanol | 10,00 |
| Carbomer | 0,70 |
| Triglycerid, flüssig | 1,50 |
| Glycerin | 5,00 |
| Panthenol | 0,50 |
| Tocopherolacetat | 0,50 |
| Desferrioxamin E | 0,10 |
| Silica | 15,00 |
| Polymethylsilsequioxan | 10,00 |
| HDI / Trimethylol Hexyllactone Crosspolymer | 5,00 |
| Eisenoxide | 2,00 |
| Titandioxid | 1,00 |
| Perlglanzpigmente Parfüm, Konservierungsmittel, NaOH, | 1,00 |
| Farbstoffe, Antioxidantien etc. | q.s. |
| Wasser | ad 100,00 |

**Beispiel 2 (dekoratives Puder)**

| | Gew. % |
|---|---|
| Carbopol | 0,50 |
| Magnesium Aluminium Silikate | 0,25 |
| PEG-5 Soja Sterole | 0,20 |
| Glycerin | 3,00 |
| Triethanolamin | 0,50 |
| Eusolex T2000 | 4,00 |
| Dimethicone / Vinyl Dimethicone Crosspolymer | 8,00 |
| Polymethylsilsesquioxane | 7,50 |
| Silica Silylate | 10,00 |
| Nylon-12 | 5,00 |
| PMMA | 2,00 |
| Eisenoxide | 2,00 |
| Titandioxid | 1,00 |
| Perlglanzpigmente | 5,00 |
| Antioxidantien, Konservierungsmittel, Parfum, Farbstoffe | q.s. |
| Wasser | ad 100,00 |

**Beispiel 3 (Faltenreduzierendes Puder):**

| | Gew. % |
|---|---|
| Xanthan Gum | 0,20 |

(fortgesetzt)

| | Gew. % |
|---|---|
| Carbomer | 0,50 |
| Glycerin | 0,50 |
| 1,3 Butylenglycol | 0,20 |
| Ubichinon (Coenzym Q10) | 0,20 |
| Iminodibernsteinsäure | 0,20 |
| Aerosil | 15,00 |
| Bornitrid | 5,00 |
| Ronaspheren LDP | 5,00 |
| Dimethicone | 2,00 |
| Perlglanzpigmente Parfüm, Konservierungsmittel, NaOH, | 3,50 |
| Antioxidantien, etc. | q.s. |
| Wasser | ad 100,00 |

**Beispiel 4 (Sonneschutzpuder):**

| | Gew. % |
|---|---|
| Xanthan Gum | 0,20 |
| Guar Gum | 0,20 |
| Magnesium Aluminium Silicate | 1,50 |
| Glycerin | 5,00 |
| Panthenol | 2,50 |
| Phenylbenzimidazol Sulfonsäure | 2,50 |
| mikronisiertes Titandioxid | 5,00 |
| Aerosil | 12,00 |
| Polymethylsilsesquioxane | 12,00 |
| Bornitrid | 5,00 |
| Aluminium Starch Octenylsuccinate (Dry Flo PC) | 2,00 |
| Farbpigmente | 8,00 |
| Parfüm, Konservierungsmittel, NaOH, Farbstoffe, Antioxidantien, etc. | q.s. |
| Wasser | ad 100,00 |

**Beispiel 5 (Puder, tonisierend):**

| | Gew. % |
|---|---|
| Xanthan Gum | 0,40 |
| Chitosan | 0,50 |
| Milchsäure (90%ig) | 0,30 |
| Ethanol | 5,00 |
| Glycerin | 2,00 |
| Polysilikon-11 | 10,00 |
| HDI / Trimethylol Hexyllactone Crosspolymer | 15,00 |
| Aerosil | 5,00 |
| Polyurethan | 2,00 |
| Perlglanzpigmente | 10,00 |

(fortgesetzt)

| | Gew. % |
|---|---|
| Cyclomethicone Parfüm, Konservierungsmittel, NaOH, | 2,00 |
| Farbstoffe, Antioxidantien, etc. | q.s. |
| Wasser | ad 100,00 |

**Beispiel 6 (mattierendes Puder):**

| | Gew. % |
|---|---|
| Carbomer | 0,35 |
| Hyroxyproylethyl Cellulose | 0,35 |
| Dimethicone | 1,50 |
| Sorbit | 2,00 |
| Ubichinon (Coenzym Q10) | 0,30 |
| Amiogum 23 (Starch Octenylsucciant) | 5,00 |
| Dimethicone / Vinyl Dimethicone Crossspolymer | 12,00 |
| Dimethicone / Phenyl Vinyl Dimethicone Crossspolymer | 4,00 |
| Dmethiconol / Silsesquioxane Copolymer Parfüm, Konservierungsmittel, Triethanolamine, | 10,00 |
| Farbstoffe, Antioxidantien, Pigmente etc. | q.s. |
| Wasser | ad 100,00 |

**Beispiel 7 (hauttönungsverstärkendes Puder):**

| | Gew. % |
|---|---|
| HDI / Trimethylol Hexyllactone Crosspolymer | 15,00 |
| Polymethylsilsesquioxane | 12,00 |
| Dimethicone | 1,50 |
| Natrium Hyaluronate | 1,00 |
| Siloxane | 1,00 |
| Silica Silylate | 1,00 |
| Natrium Polyacrylate | 1,00 |
| Perlglanzpigmente | 10,00 |
| Titandioxid | 2,00 |
| Eisenoxide Parfüm, Konservierungsmittel, Triethanolamine, | 3,00 |
| Farbstoffe, Antioxidantien, etc. | q.s. |
| Wasser | ad 100,00 |

**Beispiel 8 (Eye Shadow Puder):**

| | Gew. % |
|---|---|
| Perlglanzpigmente | 15,00 |
| Iron Oxides | 5,00 |
| Silica | 2,00 |
| Carbomer | 1,00 |
| Citric Acid | q.s. |

(fortgesetzt)

| | Gew. % |
|---|---|
| Glycerin | 5,00 |
| PVP / VA Copolymer | 2,00 |
| Triethanolamine | 1,20 |
| Dimethicone / Vinyl Dimethicone Crossspolymer Parfüm, Konservierungsmittel,Farbstoffe, | 12,00 |
| Antioxidantien, etc. | q.s. |
| Wasser | ad 100,00 |

**Beispiel 9 (Pickelpuder):**

| | Gew. % |
|---|---|
| Bornitrid | 10,00 |
| Polyacrylamide, C13-14 Isoparaffin, Laureth-7 (Seppigel 305) | 2,50 |
| 1,3 Butylenglycol | 6,00 |
| Salicylsäure | 1,25 |
| Nylon-12 | 6,00 |
| Polymethylsilsesquioxane Parfüm, Konservierungsmittel, NaOH, | 12,00 |
| Farbstoffe, Antioxidantien, Pigmente etc. | q.s. |
| Wasser | ad 100,00 |

**Patentansprüche**

**1.** Kosmetische oder dermatologische Puderzubereitungen, enthaltend

a) Wasser,
b) mindestens ein Hydrokolloid und
c) mindestens einen hydrophoben oder hydrophob modifizierten partikulären Stoff.

**2.** Zubereitung nach Anspruch 1, **dadurch gekennzeichnet, daß** der Wasserphasenanteil aus dem Bereich von 5 bis 90 Gew.-% gewählt wird, bezogen auf das Gesamtgewicht der Zubereitungen.

**3.** Zubereitung nach einem der Ansprüche 1 oder 2, **dadurch gekennzeichnet, daß** der Anteil des Hydrokolloids kleiner 10 Gew.-% gewählt wird, bezogen auf das Gesamtgewicht der Zubereitungen.

**4.** Zubereitung nach einem der vorhergehenden Ansprüche, **dadurch gekennzeichnet, daß** der Anteil an hydrophoben oder hydrophob modifizierten partikulären Stoffen (eine oder mehrere Verbindungen) größer 0,05 Gew.-% gewählt wird, bezogen auf das Gesamtgewicht der Zubereitungen.

**5.** Zubereitung nach einem der vorhergehenden Ansprüche, **dadurch gekennzeichnet, daß** das oder die Hydrokolloide gewählt werden aus der Gruppe der Cellulosederivate, insbesondere aus der Gruppe, die gebildet wird aus (Hydroxypropyl)methylcellulosen, Natriumcarboxymethylcellulose und Xanthan Gummi und/oder aus der Gruppe der Acrylat-Alkylacrylat-Copolymere und der Copolymere aus C10-30-Alkylacrylaten und einem oder mehreren Monomeren der Acrylsäure, der Methacrylsäure oder deren Ester.

**6.** Zubereitung nach einem der vorhergehenden Ansprüche, **dadurch gekennzeichnet, daß** der oder die hydrophoben oder hydrophob modifizierten partikulären Stoffe gewählt werden aus der Gruppe, welche gebildet wird aus modifizierten Polysacchariden, mikrofeinen Polymerpartikeln, Bornitrid und anorganischen Pigmenten, die gewählt werden aus der Gruppe der hydrophob beschichteten Metalloxide, insbesondere aus der Gruppe Titandioxid, Zinkoxid, Eisenoxide bzw. Eisenmischoxide, Siliciumdioxid bzw. Silicate, wobei die Pigmente sowohl einzeln als auch im Gemisch vorliegen können.

**7.** Zubereitung nach einem der vorhergehenden Ansprüche, **dadurch gekennzeichnet, daß** sie in Form eines Make-up- und/oder Gesichtspuders vorliegt und zusätzlich mindestens einen Farbstoff und/oder ein Farbpigment enthält.

**8.** Zubereitung nach einem der vorhergehenden Ansprüche, **dadurch gekennzeichnet, daß** sie in Form eines Antifaltenpuders vorliegt und mindestens einen Wirkstoff enthält, der gegen extrinsische oder intrinsische Hautalterung, Falten, Fältchen und/oder Hautschlaffheit wirkt.

**9.** Zubereitung nach Anspruch 9, **dadurch gekennzeichnet, daß** der oder die Wirkstoffe gewählt werden aus der Gruppe, welche gebildet wird aus Ubichinon und Ubichinol und deren Derivaten, Vitamin A und Derivaten (Vitamin-A-palmitat), Rutinsäure und deren Derivaten, α-Glycosylrutin und α-Hydroxycarbonsäuren und deren Salzen.

**10.** Zubereitung nach einem der vorhergehenden Ansprüche, **dadurch gekennzeichnet, daß** sie einen oder mehrere Zusatz- oder Wirkstoffe aus der Gruppe der Adstringentien und/oder der Antioxidantien und/oder der UV-Filtersubstanzen und/oder der antimikrobiell wirksamen Stoffe und/oder der gegen Akne wirksamen Stoffe enthält.

**11.** Verfahren zur Herstellung von Puderzubereitungen, **dadurch gekennzeichnet, daß** mit einem oder mehreren Hydrokolloiden in an sich bekannter Weise aus Wasser ein Gel gebildet wird, gewünschtenfalls ein oder mehrere hydrophile Wirkstoffe sowie weitere kosmetische oder pharmazeutische Hilfs-, Zusatz- und/oder Wirkstoffe zugegeben werden, und die Gelphase anschließend mit mindestens einer Sorte hydrophober oder hydrophob modifizierter Partikel versetzt wird und gewünschtenfalls weitere (färbende) Pigmente und/oder Füllstoffe hinzugefügt werden.

**12.** Kosmetisches Puderset, **dadurch gekennzeichnet, daß** eine Puderzubereitung, welche Wasser, mindestens ein Hydrokolloid und mindestens ein hydrophobes Pigment enthält, in einem wiederverschließbaren Behälter derart aufbewahrt wird, daß die Puderzubereitung nicht austrocknet.